# EUROPEAN PATENT SPECIFICATION

(11) **EP 0 557 954 B1**
(45) Date of publication and mention of the grant of the patent: **07.01.1999**
(21) Application number: 93102851.8
(22) Date of filing: 24.02.1993
(51) Int. Cl.: C12P 21/02, C12N 11/02

(54) **A process for producing epsilon-poly-L-lysine**
Verfahren zur Herstellung von Epsilon-poly-L-Lysin
Procédé pour la production de la epsilon-poly-L-lysine

(30) Priority: 26.02.1992 JP 75484/92
(43) Date of publication of application: 01.09.1993
(73) Proprietor: CHISSO CORPORATION, Osaka (JP)
(72) Inventor: Hiraki, Jun, Yokohama-shi, Kanagawa-ken (JP); Suzuki, Eriko, Yokohama-shi, Kanagawa-ken (JP)
(74) Representative: Kraus, Walter, Dr.

(56) References cited:
- EP-A- 0 256 423
- GB-A- 2 160 870
- GB-A- 2 220 946
- Rehm, H.-J. & Reed, G., "Biotechnology, vol. 7a", 1987, VCH Verlagsgesellschaft mbH, Weinheim, Germany

## Description

### BACKGROUND OF THE INVENTION

### Field of the Invention

This invention relates to a process for producing ε-poly-L-lysine using immobilized bacterial cells.

### Description of the Related Art

ε-Poly-L-lysine has already been known to be obtained by cultivating e.g. Streptomyces albulus subsp. lysinopolymerus No. 346 (JP 53-72896).

The above substance is a homopolymer of L-lysine, that is, a high molecular weight compound obtained by binding an amino group at ε-position of L-lysine to a carboxyl group of L-lysine adjacent thereto on the linear chain by means of a peptide bonding.

Since the above substance is a polymer of L-lysine which is an essential amino acid, it has a high safety, and since it has a high cation content, it has specific physical properties. Thus, by making use of these properties, there have been developed various use applications to e.g. toiletries, cosmetic preparations, feed additives, pesticides, food additives, electronic materials, etc.

Conventional process for producing ε-poly-L-lysine has been carried out as follows:

Bacterial cells belonging to Streptomyces genus having ε-poly-L-lysine productivity are cultivated under aerobic conditions, followed by adjusting the pH to that in the vicinity of 4 after the growth of the bacterial cells have been confirmed, continuing the cultivation, separating the bacterial cells from the culture solution containing ε-poly-L-lysine by means of centrifuge or filtration and purifying the bacterial cells by means of e.g. a basic anionic exchange resin treatment (JP 02-020295) or a cationic exchange resin treatment (JP 02-092927).

However, according to such a conventional process of cultivating bacterial cells of Streptomyces genus having ε-poly-L-lysine productivity under aerobic conditions and accumulating ε-poly-L-lysine in the culture solution, the viscosity of the culture solution becomes high and the bacterial cells cause bacteriolysis and hence it is impossible to reuse the bacterial cells. Further, when centrifugal separation or filtration is carried out in order to separate the bacterial cells, the bacterial cells have caused bacteriolysis and hence a long time is required.

For example, for removing the bacterial cells from a culture solution of 10 m³ using a filtration apparatus having a filtration area of 10 m², about 32 hours are required; hence such a long time has caused a serious hindrance to production. Still further, since the bacterial cells cause bacteriolysis, recovery of only the culture solution is difficult; hence it is difficult to carry out a semi-continuous cultivation wherein, for example, a fresh medium is added. The above factors hinder reduction in the production cost of ε-poly-L-lysine.

Thus, if it is possible to inhibit higher viscosity of the culture solution due to bacterial cells and the bacteriolysis of bacterial cells, it is possible to reduce the production cost of ε-poly-L-lysine. The inventors have made extensive research in a process for achieving the above object. As a result, the inventors have found that when a microorganism having the productivity of ε-poly-L-lysine is immobilized,the above-mentioned problems are solved at a stroke, and have achieved the present invention.

As apparent from the foregoing, the object of the invention is to provide a novel and efficient process for producing ε-poly-L-lysine wherein when ε-poly-L-lysine is produced using a microorganism,immobilized bacterial cells are used, whereby higher viscosity of the culture solution and bacteriolysis of the bacterial cells are inhibited, the recovery of the culture solution containing ε-poly-L-lysine and the purification of ε-poly-L-lysine from the recovered culture solution become easy, and the separated, immobilized bacterial cells are repeatedly used and semicontinuously or continuously used as the catalyst for producting ε-poly-L-lysine. In addition, the semicontinuous production referred to herein means a production wherein a medium containing a substrate or materials is added to a reactor containing the immobilized bacterial cells and the total quantity of this medium is exchanged by a fresh medium each a definite time, whereby ε-poly-L-lysine is repeatedly produced.

### SUMMARY OF THE INVENTION

The present invention has the following constitutions (1), (2), (3) and (4):
(1) Process for producing ε-poly-L-lysine, which comprises cultivating a microorganism having ε-poly-L-lysine productivity under aerobic conditions, wherein (i) said microorganism is immobilized according to a method selected from the group consisting of adsorption method, cross-linking method and entrapping method, (ii) the immobilization carrier is other than acidic high molecular weight gels and (iii) the pH at the time of cultivation of the microorganism is 4 to 7.
(2) Process for producing ε-poly-L-lysine according to item (1), which process is carried out semicontinuously or continuously.
(3) Process for producing ε-poly-L-lysine according to item (1) or (2), wherein said microorganism having ε-poly-L-lysine productivity is a bacterium belonging to Streptomyces genus.
(4) Process for producing ε-poly-L-lysine according to item (1) or (2), wherein said microorganism having ε-poly-L-lysine productivity is immobilized according to a combination of adsorption method with cross-linking method or a combination of adsorption method with entrapping method.

### DETAILED DESCRIPTION OF PREFERRED EMBODIMENTS

As the microorganisms used in the present invention, any of microorganisms having ε-poly-L-lysine productivity may be used.

As such microorganisms, for example, Streptomyces abulus subsp. lysinopolymerus No. 346 (JP 53-72896), variant producing ε-poly-L-lysine in a notably large quantity, No. 11011A-1 (JP 63-49075), Streptomyces noursei (JP 1-187090), etc. have been known.

Further, the immobilization of the producing bacterium used in the present invention can be carried out according to a general method or application of a general method. For example, adsorption method, crosslinking method, entrapping method, covalent bond method may be used.

The immobilized bacterial cells are prepared according to a single immobilization method. Further, an immobilization method can be carried out in a combination of the above two methods. Among the above methods, adsorption method and entrapping method are preferably employed, since according to the methods, the immobilization is easy and a stabilized activity is obtained and further, when the activity has lowered, reactivation can be carried out. The entrapping method is generally carried out by entrapping the bacterial cells in a high molecular weight polymer gel such as polyacrylamide gel, polyurethane gel, photo-crosslinkable resin. In the present invention, however, since ε-poly-L-lysine to be produced is a basic substance, it is preferred not to use an acidic high molecular weight compound gel which adsorbs the substance, such as carrageenan gel, alginic acid gel.

As the adsorption method, a method of immobilization onto a porous substance or a non-woven cloth is particularly preferred. As the porous substance, for example, porous ceramics, porous glass, cellulose sponge, sintered metal porous body, those prepared from polyvinyl chloride, polyethylene, polypropylene, polystyrene, polyurethane, etc. may also be used.

As the above entrapping in polyacrylamide gel, for example, acrylamide monomer, N,N'-methylenebisacrylamide as a crosslinking agent and live bacterial cells are suspended in a buffer, followed by adding ammonium persulfate as a polymerization initiator and β-dimethylaminopropionitrile as a polymerization promotor to the buffer and subjecting the mixture to polymerization reaction at room temperature for about 30 minutes to obtain immobilized bacterial cells,

Further, in general, when thermoplastic resins are heated at a temperature of 150° to 250°C for 0.5 to 5 hours, sintered bodies are obtained, and when a suitable mold is used at the time of the heating, sintered porous bodies having various shapes are obtained. Still further, as to voids, pore size, strength, etc., when conditions of sintering temperature, sintering time, packing of resin, thickening agent, quantity of water added, etc. are varied, preferred porous bodies are obtained. As other porous substances than the above, any of general commercially available products and processed products may be used as far as they can adsorb the present producing bacteria.

The porous substances are immersed in a buffer containing live bacterial cells to adsorb the microorganism, or the porous substance is contacted with a microorganism since the initiation time of the cultivation and adhesion is effected along with advance of the cultivation, whereby immobilized bacterial cells according to the process of the present invention are obtained.

The immobilized bacterial cells having ε-poly-L-lysine productivity obtained according to the above process arecultivated in a usual ε-poly-L-lysine-producing medium or a medium having added glucose and ammonium sulfate or L-lysine into a buffer adjusted to an adequate pH, under aerobic conditions, to obtain a culture solution containing ε-poly-L-lysine. The immobilized bacterial cells are removed from the culture solution and ε-poly-L-lysine is purified with an ion exchange resin.

Further, the pH at the time of cultivation may fall within a range wherein the used microorganism having ε-poly-L-lysine productivity can produce ε-poly-L-lysine. Preferable pH is 4 to 7.

According to the process of the invention, no bacteriolysis of the bacterial cells during the cultivation occurs, the immobilized bacterial cells are easily removed, and a ε-poly-L-lysine solution containing no bacterial cells can be recovered in a short time, whereby an efficient production of poly-L-lysine becomes possible. Further, since it is easy to separate the immobilized bacterial cells from the culture solution, the culture solution is recovered after production of ε-poly-L-lysine and a fresh medium is added to the immobilized bacterial cells, whereby a semi-continuous production of ε-poly-L-lysine can be carried out, or a fresh medium is added while continuously discharging the culture solution, whereby a continuous production of ε-poly-L-lysine can be carried out. Thus, the invention is very useful in that ε-poly-L-lysine can be produced effectively and commercially, at a low cost and over a long time and can be fed to the fields of food additives, pesticides, pharmaceuticals, etc.

### Example 1

Into a 500 mℓ capacity shaking flask were placed a medium (pH 6.8, 50 mℓ) consisting of glucose (50 g/ℓ), ammonium sulfate (10 g/ℓ), yeast extract (5 g/ℓ), potassium dihydrogenphosphate (1.36 g/ℓ), sodium monohydrogenphosphate (1.58 g/ℓ), magnesium sulfate (0.5 g/ℓ), zinc sulfate (0.04 g/ℓ) and ferrosulfate (0.03 g/ℓ), followed by sterilizing the medium in a conventional manner, planting Streptomyces albulus, cultivating it under shaking at 30°C for 36 hours to obtain a pre-culture solution, subjecting it, after the cultivation, to centrifugal separation to collect the bacterial cells, twice washing the bacterial cells (wet weight of bacterial cells: 4.2 g) with sterilized water and using the resulting cells for immobilization.

Preparation of immobilized bacterial cells was carried out as follows:

Acrylamide (1.68 g), N,N'-methylenebisacrylamide (0.09 g), lysine hydrochloride (0.67 g) and the bacterial cells obtained according to the above method were suspended in tris-hydrochloric acid buffer (pH: 7.2, 4.8 mℓ) (tris: 2-amino-2-hydroxymethyl-1,3-propanediol), followed by adding to the mixed solution, a 5% aqueous solution of β-dimethylaminopropionitrile (1.1 mℓ) and a 1% aqueous solution of potassium persulfate (1.1 mℓ), allowing the mixture to stand still under N₂-saturated condition, at room temperature for 30 minutes to effect gelation, shaping the gel into 5 mm cube , and sufficiently washing the resulting gel with Tris-hydrochloric acid buffer to obtain bacterial cells immobilized in polyacrylamide gel (11.2 g).

The resulting bacterial cells immobilized in the polyacrylamide gel were added into a medium (pH 4, 40 mℓ) consisting of glucose (50 g/ℓ), L-lysine (10 g/ℓ) and citric acid (20 g/ℓ), followed by cultivation under shaking at 30°C. After 2 days, the content of £-poly-L-lysine in the culture solution was determined to give 0.23 mg/mℓ.

### Example 2

Bacterial cells immobilized in polyacrylamidegel(75g), prepared in the same manner as in Example 1 were added into a conventional medium for cultivating ε-poly-L-lysine-producing bacterium (pH 4.2, 50 mℓ) consisting of glucose (50 g/ℓ), ammonium sulfate (10 g/ℓ), yeast extract (5 g/ℓ), potassium dihydrogenphosphate (1.36 g/ℓ), disodium monohydrogenphosphate (1.58 g/ℓ), magnesium sulfate (0.5 g/ℓ), zinc sulfate (0.04 g/ℓ) and ferrosulfate (0.03 g/ℓ), followed by cultivation under shaking at 30°C. After 2 days, the content of ε-poly-L-lysine in the culture solution was 0.20 mg/mℓ.

### Example 3

Bacterial cells immobilized in polyacrylamide gel(100g) prepared in the same manner as in Example 1 were added into a medium (pH 4, 1 ℓ) consisting of glucose (50 g/ℓ), L-lysine (10 g/ℓ) and citric acid (20 g/ℓ), followed by through-flow stirring cultivation at 30°C by means of a 1.5 ℓ capacity minijar fermenter while adjusting the pH to 4. The content of ε-poly-L-lysine in the culture solution after cultivation for 48 hours was 1.5 g/ℓ.

Thereafter, cultivation was continued while successively adding glucose and L-lysine. The content of ε-poly-L-lysine after 125 hours was 10 g/ℓ. The culture solution was subjected to centrifugal separation (3,000 G, 20 minutes) to remove the bacterial cells, followed by measuring the absorbance of the supernatant after the centrifugal separation at 660 nm. The absorbance was 0.008 and the bacterial cells were completely removed.

### Comparative example 1

Into a 500 mℓ capacity shaking flask were placed a medium (pH 6.8, 50 mℓ) consisting of glucose (50 g/ℓ), ammonium sulfate (10 g/ℓ), yeast extract (5 g/ℓ), potassium dihydrogenphosphate (1.36 g/ℓ), sodium monohydrogenphosphate (1.58 g/ℓ), magnesium sulfate (0.5 g/ℓ), zinc sulfate (0.04 g/ℓ) and ferrosulfate (0.03 g/ℓ), followed by sterilizing the mixture in a conventional manner, inoculating Streptomyces albulus and cultivating under shaking at 30°C for 36 hours to prepare a pre-culture solution.

This pre-culture solution was planted in a 1.5 ℓ capacity minijar fermenter containing the same medium as the above (1 ℓ), followed by through-flow stirring cultivation at 30°C. After the cultivation, when the pH of the culture solution lowered down to about 4.2, glucose was successively added so as to give a concentration of 50 g/ℓ, while adjusting its pH to 4.2 with aqueous NaOH solution, and continuing the cultivation.

The content of ε-poly-L-lysine in the culture solution after 125 hours was 12 g/ℓ. This culture solution was subjected to centrifugal separation (3,000G, 20 minutes) to remove the bacterial cells and measuring the absorbance of the supernatant at 660 nm. The absorbance was 0.282, so that the removal of the bacterial cells was insufficient.

From the above results, it was confirmed to be able to easily remove the bacterial cells by using the immobilized bacterial cells of the present invention.

### Example 4

Bacterial cells (wet weight: 2.0 g) cultivated and recovered in the same manner as in Example 1 were suspended in a 0.1 M phosphoric acid buffer (2 mℓ), followed by adding and mixing an urethane prepolymer (3 g, M.W.: about 3,000) having a structure shown in formula 1, with stirring and effecting gelation to obtain immobilized bacterial cells of polyurethane gel shaped into 5 mm cube.

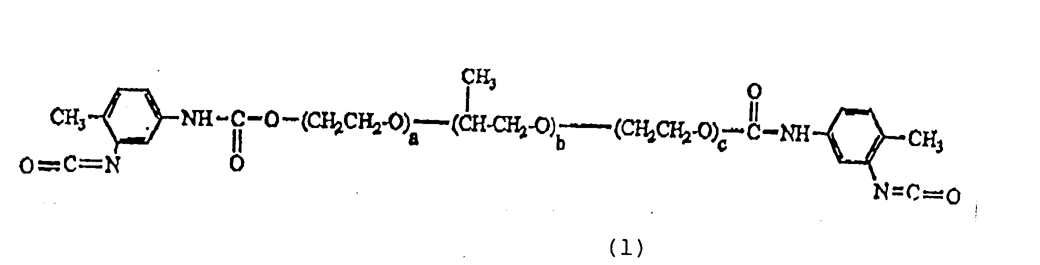

The bacterial cells immobilized in the polyurethane gel were added to a medium (pH 4, 40 mℓ) containing glucose (50 g/ℓ), 1-lysine (10 g)/ℓ and citric acid (20 g/ℓ), followed by cultivating the mixture under shaking at 30°C and measuring the quantity of ε-poly-L-lysine in the culture solution after 2 days and 3 days. The quantities of ε-poly-L-lysine in the culture solution were 0.31 g/ℓ after 2 days and 0.47 g/ℓ after 3 days.

### Example 5

Cultivation under the same conditions as in Example 4 was carried out for 3 days, followed by removing the supernatant obtained by centrifugal separation (3,000G,20 minutes), adding a fresh medium (pH: 4, 40 mℓ) consisting of glucose (50 g/ℓ), L-lysine (10 g/ℓ) and citric acid (20 g/ℓ) and cultivating the mixture under shaking at 30°C. After lapse of 2 days after having added the fresh medium, the quantity of ε-poly-L-lysine in the culture solution was 0.75 mg/mℓ.

From the above results, it was confirmed that the immobilized bacterial cells of the present invention could be applied to semi-continuous cultivation.

### Example 6

Bacterial cells (wet weight: 2.0 g) cultivated and recovered in the same manner as in Example 1 were suspended in sterilized water (5 mℓ), followed by adding and mixing 15% aqueous solution (40 mℓ) of a photo-crosslinkable resin ENT-2000 (tradename of product made by Kansai Paint Co., Ltd.) sterilized in an autoclave and a polymerization initiator (0.008 g), and then irradiating ultraviolet rays (365 nm) upon the resulting suspension with stirring for 30 minutes, to prepare a gel having immobilized bacterial cells. This gel was shaped into 5 mm cubic to obtain fixed bacterial cells (53 g).
The immobilized bacterial cells in the photo-crosslinkable resin were added to a medium (pH: 4, 100 mℓ) containing glucose (50 g/ℓ), 2-lysine (10 g/ℓ) and citric acid (20 g/ℓ), followed by cultivating the cells under shaking at 30°C and measuring the quantities of ε-poly-L-lysine in the culture solution after 2 days and 3 days to give 0.44 g/ℓ after 2 days and 0.57 g/ℓ after 3 days.

### Example 7

The bacterial cells precultivated in the same manner as in Example 1 were recovered by means of centrifugal separation and washed with sterilized water (wet bacterial cells weight: 4.0 g). Into a medium (pH: 6.8, 30 mℓ) consisting of glucose (50 g/ℓ), ammonium sulfate (10 g/ℓ), yeast extract (5 g/ℓ), potassium dihydrogenphosphate (1.36 g/ℓ), disodium monohydrogenphosphate (1.58 g/ℓ), magnesium sulfate (0.5 g/ℓ), zinc sulfate (0.04 g/ℓ) and ferrosulfate (0.03 g/ℓ), were placed the above bacterial cells and further a polyurethane sintered porous beads ( size : ⌀ 5mm, 2 g), followed by cultivation under slow shaking at 30°C.

After 30 hours, bacterial cells immobilized in polyurethane sintered porous body were recovered. The immobilized bacterial cells were suspended in a medium (pH: 4, 50 mℓ) containing glucose (50 g/ℓ), L-lysine (10 g/ℓ) and citric acid (20 g/ℓ), followed by cultivation under shaking at 30°C. The quantity of ε-poly-L-lysine in the culture solution after 2 days of the cultivation was 0.62 mg/mℓ.

Further, the culture solution was subjected to centrifugal separation (3,000G, 20 minutes) and the absorbance of the resulting supernatant was measured at 660 nm. The absorbance was 0.009 and the bacterial cells were completely removed.

### Example 8

Into a 400 mℓ capacity air bubble-through type reactor were fed a medium (pH 6.8, 100 mℓ) consisting of glucose (50 g/ℓ), ammonium sulfate (10 g/ℓ), yeast extract (5 g/ℓ), potassium dihydrogen phosphate (1.36 g/ℓ), disodium monohydrogen phosphate (1.58 g/ℓ), magnesium sulfate (0.5 g/ℓ), zinc sulfate (0.04 g/ℓ) and ferrosulfate (0.03 g/ℓ), and a block-form ceramic (made by Nihon Gaishi Co., Ltd.) (30 g), followed by sterilizing them in a conventional manner, incubating Streptomyces albulus taken from a slant medium for preserving bacterial cells in a quantity of one platinum loop, and carrying out aeration cultivation in an aeration quantity of 0.5 ℓ/min. at 30°C for 50 hours. The quantity of ε-poly-L-lysine in the culture solution after 50 hours was 0.3 g/ℓ. Thereafter, only the culture solution was recovered, followed by adding to the immobilized bacterial cells in the reactor, a fresh medium (pH 4, 100 mℓ) consisting of glucose (50 g/ℓ), L-lysine hydrochloride (10 g/ℓ), citric acid (20 g/ℓ), potassium dihydrogen sulfate (1.36 g/ℓ), sodium monohydrogen phosphate (1.58 g/ℓ), magnesium sulfate (0.5 g/ℓ), zinc sulfate (0.04 g/ℓ) and ferrosulfate (0.03 g/ℓ), and carrying out aeration cultivation at 30°C for 48 hours. The quantity of ε-poly-L-lysine in the culture solution after 48 hours was 3.7 g/ℓ. Further, the culture solution was recovered, followed by adding a fresh medium and three times repeating a procedure of carrying out aeration cultivation at 30°C for 48 hours. As a result, the quantities of ε-poly-L-lysine at the respective repeated times were as follows:
4.5 g/ℓ (first time), 4.0 g/ℓ (second time) and
5.2 g/ℓ (third time).

From the foregoing, it was confirmed that when medium exchange was repeated using the immobilized bacterial cells of the present invention, the semi-continuous production of ε-poly-L-lysine was effected.

### Example 9

In a 400 mℓ capacity air bubble-through type reactor were placed a medium (pH 6.8, 100 mℓ) consisting of glucose (50 g/ℓ), ammonium sulfate (10 g/ℓ), yeast extract (5 g/ℓ), potassium dihydrogen phosphate (1.36 g/ℓ), disodium monohydrogen phosphate (1.58 g/ℓ), magnesium sulfate (0.5 g/ℓ), zinc sulfate (0.04 g/ℓ) and ferrosulfate (0.03 g/ℓ), and a cellulose sponge (Microcube FN-S03 (trademark of product made by Sakai Engineering Co., Ltd.)), followed by sterilizing the medium in a conventional manner, inoculating Streptmyces albulus taken from a slant medium for preserving bacterial cells in a quantity of one platinum loop, and carrying out aeration cultivation at 30°C for 50 hours in an aeration quantity of 0.5 ℓ/min. The quantity of ε-poly-L-lysine in the culture solution after 50 hours was 0.33 g/ℓ. Thereafter, only the culture solution was recovered, followed by adding to the fixed bacterial cells in the reactor, a fresh medium (pH 4, 100 mℓ) consisting of glucose (50 g/ℓ), L-lysine hydrochloride (10 g/ℓ), citric acid (20 g/ℓ), potassium dihydrogen phosphate (1.36 g/ℓ), sodium monohydrogen phosphate (1.58 g/ℓ), magnesium sulfate (0.5 g/ℓ), zinc sulfate (0.04 g/ℓ) and ferrosulfate (0.03 g/ℓ), and carrying out aeration at 30°C for 48 hours. The quantity of ε-poly-L-lysine in the culture solution after 48 hours was 2.9 g/ℓ. Further, the culture solution was recovered, followed by adding a fresh medium and three times repeating a procedure of carrying out aeration cultivation at 30°C for 48 hours. As a result, the quantities of ε-poly-L-lysine produced at the respective repetition times were as follows:
3.0 g/ℓ (first time), 2.5 g/ℓ (second time) and
2.0 g/ℓ (third time).

From the foregoing, it was confirmed that when the medium exchange was repeately carried out using the immobilized bacterial cells of the present invention, a semi-continuous production of ε-poly-L-lysine was possible.

### Comparative example 2

Bacterial cells were cultivated without feeding the ceramic carrier in Example 8. Namely, in a 400 mℓ capacity air bubble-through type reactor was placed a medium (pH 6.8, 100 mℓ) consisting of glucose (50 g/ℓ), ammonium sulfate (10 g/ℓ), yeast extract (5 g/ℓ), potassium dihydrogen phosphate (1.36 g/ℓ), disodium monohydrogen phosphate (1.58 g/ℓ), magnesium sulfate (0.5 g/ℓ), zinc sulfate (0.04 g/ℓ) and ferrosulfate (0.03 g/ℓ), followed by sterilizing the medium in a conventional manner, inoculating Streptomyces albulus taken from a slant medium for preserving bacterial cells in a quantity of one platinum loop and carrying out aeration cultivation at 30°C, in an aeration quantity of 0.5 ℓ/min. for 50 hours. The quantity of ε-poly-L-lysine in the culture solution after 50 hours was 0.25 g/ℓ. Thereafter, bacterial cells were recovered from the culture solution by centrifuge (6,000 G, 15 min.), followed by adding to the recovered bacterial cells, a fresh medium (pH 4, 100 mℓ) consisting of glucose (50 g/ℓ), L-lysine hydrochloride (10 g/ℓ), citric acid (20 g/ℓ), potassium dihydrogen phosphate (1.36 g/ℓ), sodium monohydrogen phosphate (1.58 g/ℓ), magnesium sulfate (0.5 g/ℓ), zinc sulfate (0.04 g/ℓ) and ferrosulfate (0.03 g/ℓ), and carrying out aeration at 30°C, for 48 hours. The quantity of ε-poly-L-lysine in the culture solution after 48 hours was 1.3 g/ℓ. Further, bacterial cells were recovered from the culture solution by centrifuge (6,000 G, 15 min.), followed by again adding the above fresh medium and carrying out aeration cultivation at 30°C for 48 hours. The quantity of ε-poly-L-lysine in the culture solution after 48 hours was 0.7 g/ℓ.

From the above results, it was confirmed that according to semi-continuous production of ε-poly-L-lysine wherein bacterial cells were recovered without using immobilized bacterial cells and cultivation was carried out, the productivity of ε-poly-L-lysine lowered each repeated times; hence semi-continuous production was difficult.

### Example 10

In a 400 mℓ capacity air bubble-through type reactor were placed a medium (pH 6.8, 100 mℓ) consisting of glucose (50 g/ℓ), ammonium sulfate (10 g/ℓ), yeast extract (5 g/ℓ), potassium dihydrogen phosphate (1.36 g/ℓ), disodium monohydrogen phosphate (1.58 g/ℓ), magnesium sulfate (0.5 g/ℓ), zinc sulfate (0.04 g/ℓ) and ferrosulfate (0.03 g/ℓ), and a block-form ceramic (made by Nihon Gaishi Co., Ltd.) (30 g), followed by sterilizing them in a conventional manner, inoculating Streptmyces albulus taken from a slant medium in a quantity of one platinum loop and carrying out aeration cultivation at 30°C, in an aeration quantity of 0.5 ℓ/min. for 50 hours. The quantity of ε-poly-L-lysine in the culture solution after 50 hours was 0.3 g/ℓ. Thereafter, a continuous production of ε-poly-L-lysine over 300 hours was carried out wherein addition into the reactor, of a fresh medium (pH 4) consisting of glucose (50 g/ℓ), L-lysine hydrochloride (10 g/ℓ), citric acid (20 g/ℓ), potassium dihydrogen phosphate (1.36 g/ℓ), sodium monohydrogen phosphate (1.58 g/ℓ), magnesium sulfate (0.5 g/ℓ), zinc sulfate (0.04 g/ℓ) and ferrosulfate (0.03 g/ℓ) and withdrawal of the culture solution from the reactor were carried out at a rate of 2 mℓ/hr. The total quantity of ε-poly-L-lysine produced over 300 hours was 2.2 g.

From the foregoing, it was confirmed that when the medium exchange is continuously carried out using the immobilized bacterial cells, it is possible to easily carry out a continuous production of ε-poly-L-lysine.

## Claims

1. Process for producing ε-poly-L-lysine, which comprises cultivating a microorganism having ε-poly-L-lysine productivity under aerobic conditions, wherein (i) said microorganism is immobilized according to a method selected from the group consisting of adsorption method, cross-linking method and entrapping method, (ii) the immobilization carrier is other than acidic high molecular weight gels and (iii) the pH at the time of cultivation of the microorganism is 4 to 7.

2. Process for producing ε-poly-L-lysine according to claim 1, which process is carried out semicontinuously or continuously.

3. Process for producing ε-poly-L-lysine according to claim 1 or 2, wherein said microorganism having ε-poly-L-lysine productivity is a bacterium belonging to Streptomyces genus.

4. Process for producing ε-poly-L-lysine according to claim 1 or 2, wherein said microorganism having ε-poly-L-lysine productivity is immobilized according to a combination of adsorption method with cross-linking method or a combination of adsorption method with entrapping method.

## Patentansprüche

1. Verfahren zur Herstellung von ε-Poly-L-lysin durch Kultivieren eines Mikroorganismus mit ε-Poly-L-lysin-Produktivität bei aeroben Bedingungen, wobei (i) der Mikroorganismus gemäß einem Verfahren immobilisiert ist, ausgewählt aus der Gruppe bestehend aus einem Adsorptionsverfahren , einem Vernetzungsverfahren und einem Einschluß- bzw. Einfangverfahren, (ii) der Immobilisierungsträger ein anderer ist als saure Gele mit hohem Molekulargewicht und (iii) der pH zum Zeitpunkt der Kultivierung des Mikroorganismus 4 bis 7 beträgt.

2. Verfahren zur Herstellung von ε-Poly-L-lysin gemäß Anspruch 1, dadurch **gekennzeichnet**, daß das Verfahren semikontinuierlich oder kontinuierlich durchgeführt wird.

3. Verfahren zur Herstellung von ε-Poly-L-lysin nach Anspruch 1 oder 2, dadurch **gekennzeichnet**, daß der Mikroorganismus mit ε-Poly-L-lysin-Produktivität ein Bakterium ist, das zum Streptomyces-Genus gehört.

4. Verfahren zur Herstellung von ε-Poly-L-lysin gemäß Anspruch 1 oder 2, dadurch **gekennzeichnet**, daß der Mikroorganismus mit ε-Poly-L-lysin-Produktivität gemäß einer Kombination eines Adsorptionsverfahrens mit einem Vernetzungsverfahren oder einer Kombination des Adsorptionsverfahrens mit dem Einschluß- bzw. Einfangverfahren immobilisiert ist.

## Revendications

1. Procédé pour la production de ε-poly-L-lysine, qui comprend la culture d'un microorganisme capable de produire de la ε-polyL-lysine dans des conditions aérobies, dans lequel (i) ce microorganisme est immobilisé par un procédé choisi dans le groupe consistant en procédé d'adsorption, procédé de réticulation et procédé de piégeage, (ii) le support d'immobilisation est autre que des gels acides de haut poids moléculaire, et (iii) le pH est de 4 à 7 au moment de la culture du microorganisme.

2. Procédé pour la production de ε-poly-L-lysine suivant la revendication 1, qui est conduit de façon semi-continue ou continue.

3. Procédé pour la production de ε-poly-L-lysine suivant les revendications 1 ou 2, dans lequel ce microorganisme capable de produire de la ε-poly-L-lysine est une bactérie appartenant au genre *Streptomyces.*

4. Procédé pour la production de ε-poly-L-lysine suivant les revendications 1 ou 2, dans lequel ce microorganisme capable de produire de la ε-poly-L-lysine est immobilisé par une combinaison de procédé d'adsorption et de procédé de réticulation ou par une combinaison de procédé d'adsorption et de procédé de piégeage.
